# EUROPÄISCHE PATENTSCHRIFT

(11) **EP 3 967 350 B1**
(45) Veröffentlichungstag und Bekanntmachung des Hinweises auf die Patenterteilung: **12.06.2024**
(21) Anmeldenummer: 21000239.0
(22) Anmeldetag: 23.08.2021
(51) Int. Cl.: A61M 16/00, G16H 20/40, G16H 40/63, A61B 5/0536, A61B 5/08, G16H 20/10, G16H 40/67, G16H 50/20, G16H 50/70, A61B 5/0205, A61B 5/083, A61B 5/091, A61B 5/145, A61B 5/00, A61M 16/01

(54) **KOORDINATIONSEINHEIT UND BEHANDLUNGSSYSTEM**
COORDINATION UNIT AND TREATMENT SYSTEM
UNITÉ DE COORDINATION ET SYSTÈME DE TRAITEMENT

(30) Priorität: 10.09.2020 DE 102020123601
(43) Veröffentlichungstag der Anmeldung: 16.03.2022
(73) Patentinhaber: Löwenstein Medical Technology S.A., 2557 Luxembourg (LU)
(72) Erfinder: Kremeier, Peter, 76149 Karlsruhe (DE)
(74) Vertreter: Marx, Thomas

(56) Entgegenhaltungen:
- EP-A1- 0 753 320
- EP-B1- 2 404 253
- WO-A1-2018/085563
- US-A1- 2007 041 626
- US-A1- 2009 006 133
- US-A1- 2011 041 850
- Dimitris I. Fotiadis ET AL: "Intelligent Patient Monitoring" In: "Wiley Encyclopedia of Biomedical Engineering", 1. Januar 2006 (2006-01-01), John Wiley & Sons, Inc., Hoboken, NJ, USA, XP055427307, ISBN: 978-0-471-74036-0 DOI: 10.1002/9780471740360.ebs0642, * Seite 1, linke Spalte, Absatz 1 - Seite 2, linke Spalte, Absatz 1 *

## Beschreibung

Die Erfindung betrifft eine Koordinationseinheit für eine Gruppe von medizintechnischen Geräten und ein Behandlungssystem umfassend die Koordinationseinheit. Viele moderne medizintechnische Geräte wie beispielsweise Beatmungsgeräte erlauben die Abfrage von Daten über Computernetzwerke. Die abgefragten Daten können von medizinischem Personal zu diagnostischen Zwecken und zu Dokumentationszwecken genutzt werden.

Aus dem Stand der Technik sind Systeme zur Datenabfrage von medizintechnischen Geräten bekannt. Die Druckschrift EP 1571976 B1 beschreibt ein implantierbares Gerät, das Sensordaten empfangen kann. Bei dem implantierbaren Gerät kann es sich beispielsweise um einen Herzschrittmacher handeln. Weitere implantierte Geräte im Körper eines Patienten können an das implantierbare Gerät Sensordaten übermitteln. Das implantierbare Gerät aggregiert die Daten und übermittelt diese an eine Gegenstelle außerhalb des Körpers des Patienten. Die Druckschrift EP 2404253 B1 beschreibt ein medizinisches Überwachungssystem. Dieses kann mit verschiedenen medizinischen Sensorsystemen verbunden werden. Beim Auftreten bestimmter Sensorwerte gibt das medizinische Überwachungssystem eine Warnmeldung aus. Daraufhin kann das Klinikpersonal tätig werden.

Die Druckschrift WO 2018/085563 A1 beschreibt ein medizinisches Überwachungssystem, das einen Patienten mit Hilfe verschiedener Sensoren überwachen kann. Falls gemessene Werte vordefinierte Parameter überschreiten, kann eine Warnung ausgegeben werden oder eine Behandlung des Patienten veranlasst werden. Das Überwachungssystem kann mittels eines Rechnernetzes implementiert sein. Aus der Druckschrift US 2009/0006133 A1 ist ein Therapiesystem mit einem Nutzerinterface bekannt. Das Therapiesystem weist ein elektronisches Gerät auf, an das eine Vielzahl von medizinischen Geräten angeschlossen werden kann. Dies können beispielsweise Geräte zur Messung des Blutzuckers, des Blutdrucks oder der Körpertemperatur sein. Das elektronische Gerät kann auf Grundlage der gemessenen Werte eine Behandlung veranlassen.

Die Druckschrift EP 0 753 320 A1 betrifft ein Beatmungssystem. Das Beatmungssystem erfasst über verschiedene Sensoren Messwerte bei einem Beatmungsvorgang an einem Patienten. Die Messwerte können auf einem Bildschirm ausgegeben werden. Ein Steuerungsmodul nimmt Berechnungen auf Grundlage der Messwerte sowie ergänzender Patientendaten vor und ermittelt somit neue Ansteuerungsparameter für ein Beatmungsgerät. Der Stand der Technik kennt somit die Koordinationseinheit nach dem Oberbegriff des Anspruchs 1 sowie das Behandlungssystem nach dem Oberbegriff des Anspruchs 9.

Der Erfindung liegt die Aufgabe zugrunde, einen Entwöhnungsprozess für eine auf einer Intensivstation erworbene Muskelschwäche zu ermöglichen. Die Aufgabe wird gelöst durch die Bereitstellung einer Koordinationseinheit gemäß Oberbegriff und kennzeichnendem Teil des Anspruchs 1. Die Aufgabe wird ferner gelöst durch die Bereitstellung des Behandlungssystems gemäß Oberbegriff und kennzeichnendem Teil des Anspruchs 9.

Gemäß einem ersten Aspekt der Erfindung wird eine Koordinationseinheit für eine Gruppe von medizintechnischen Geräten vorgeschlagen. Die Koordinationseinheit ist eingerichtet zum Empfang von Sensordaten von mindestens einem der medizintechnischen Geräte, wobei die Sensordaten einem Patienten zugeordnet sind, zum Empfang von einem medizinischen Datensatz, der dem Patienten zugeordnet ist, und zur Veranlassung einer Auswertung der Sensordaten und/oder des mindestens einen medizinischen Datensatzes, um ein Behandlungsschema abzuleiten. Erfindungsgemäß verzeichnet der medizinische Datensatz, dass der Patient an einer auf einer Intensivstation erworbenen Muskelschwäche leidet, wobei die Sensordaten und/oder der medizinische Datensatz Messwerte von einem Gerät zur elektrischen Impedanz-Tomographie und/oder von einem Beatmungsgerät umfasst, wobei der medizinische Datensatz optional einen Sedierungsstatus des Patienten angibt, und wobei das Behandlungsschema aus den Sensordaten und optional aus dem mindestens einen medizinischen Datensatz abgeleitet mindestens eine für einen Entwöhnungsprozess von einem Beatmungsgerät spezifische Anweisung umfasst.

Die Sensordaten und/oder der mindestens eine medizinische Datensatz werden nicht nur ausgewertet, sondern daraus wird auch ein Behandlungsschema abgeleitet. Das Behandlungsschema kann erfindungsgemäß eine oder mehrere Handlungsanweisungen umfassen, die von einem Menschen ausgeführt werden können.

Das Behandlungsschema kann alternativ oder zusätzlich ein oder mehrere Handlungsanweisungen umfassen, die von einem medizintechnischen Gerät ausgeführt werden können. Somit kann die Koordinationseinheit in Reaktion auf einen Istzustand eines Patienten notwendige therapeutische Maßnahmen veranlassen. Die im klinischen Umfeld anfallenden Datenmengen werden ausgewertet und zur Therapie von Erkrankungen nutzbar gemacht. Es ist anzumerken, dass die Koordinationseinheit das Behandlungsschema gemäß einigen Ausführungsformen der Erfindung nicht oder zumindest nicht in allen Fällen eigenständig ableitet, sondern dazu auch die Rechenkraft entfernter Rechnersysteme nutzen kann.

Die Koordinationseinheit kann optional dazu eingerichtet sein, Patientendaten zu archivieren, die bei der Behandlung anfallen. Der medizinische Datensatz kann einen Datenwert oder eine Sammlung von Datenwerten beinhalten, die dem Patienten zugeordnet sind. So kann es sich bei dem medizinischen Datensatz beispielsweise um einen Datenbankeintrag betreffend einen Patienten, um einen Auszug aus einer elektronischen Patientenakte oder um mindestens einen durch einen Benutzer eingegebenen Wert betreffend den Patienten handeln. So kann medizinisches Personal beispielsweise der Koordinationseinheit fehlende Datenwerte ad hoc per Tastatureingabe bereitstellen. Es ist auch möglich, dass in dem medizinischen Datensatz Daten aus mehreren Datenquellen aggregiert sind, beispielsweise Datensätze aus Patientenakten und archivierte Sensordaten.

Die Koordinationseinheit ist vorzugsweise ferner dazu eingerichtet, mindestens eines der medizintechnischen Geräte so anzusteuern, dass eine Umsetzung des Behandlungsschemas erfolgt und/oder die Umsetzung des Behandlungsschemas unterstützt wird. So kann die Koordinationseinheit erfindungsgemäß Handlungsanweisungen an ein medizintechnisches Gerät übermitteln, das die Handlungsanweisungen ausführt. Zum Beispiel kann die Koordinationseinheit eine mandatorische Beatmungsfrequenz bei einem Beatmungsgerät einstellen. Es ist erfindungsgemäß ferner möglich, dass die Koordinationseinheit medizintechnische Geräte so ansteuert, dass die Umsetzung des Behandlungsschemas lediglich unterstützt wird. Dies ist dann der Fall, wenn nicht alle gemäß dem Behandlungsschema vorgesehenen Schritte von medizintechnischen Geräten ausgeführt werden können. Das Behandlungsschema kann beispielsweise vorsehen, dass eine Sedierung des Patienten notwendig ist und sich daran weitere Schritte anschließen, die nur von medizinischem Fachpersonal vorgenommen werden können. In diesem Fall veranlasst die Koordinationseinheit gemäß einer Ausführungsform der Erfindung die Aktivierung einer Spritzenpumpe mit einem Sedativ und nachfolgende Schritte des Behandlungsschemas werden ausschließlich von dem medizinischen Fachpersonal vorgenommen.

Es ist vorteilhaft, wenn die Koordinationseinheit dazu eingerichtet ist, das Behandlungsschema auszugeben, ein Bestätigungssignal zu erhalten, und nur bei Erhalt des Bestätigungssignals das mindestens eine der medizintechnischen Geräte so anzusteuern, dass die Umsetzung des Behandlungsschemas erfolgt oder die Umsetzung des Behandlungsschemas unterstützt wird. Unter der Ausgabe des Behandlungsschemas ist sowohl eine graphische Ausgabe des Behandlungsschemas zu verstehen, zum Beispiel auf einem Bildschirm, als auch eine rein technische Ausgabe des Behandlungsschemas wie eine Übermittlung des Behandlungsschemas über ein Computernetzwerk. Die Koordinationseinheit kann beispielsweise dazu eingerichtet sein, das Behandlungsschema mittels einer Ethernet-Verbindung oder einer WLAN-Verbindung an eine Gegenstelle zu übermitteln. Die Koordinationseinheit steuert das mindestens eine der medizintechnischen Geräte nur an, wenn es dazu das Bestätigungssignal erhält. So kann ein Benutzer zunächst eine Rückmeldung an die Koordinationseinheit geben, dass er das Behandlungsschema erhalten hat und mit dessen Umsetzung einverstanden ist, bevor die Koordinationseinheit weitere Schritte veranlasst. Auf diese Weise kann sichergestellt werden, dass kritische therapeutische Schritte von der Koordinationseinheit nur dann veranlasst werden, nachdem eine Überprüfung durch medizinisches Personal erfolgt ist.

Es ist besonders vorteilhaft, wenn das Behandlungsschema eine medizinische Diagnose beinhaltet. Die Diagnose kann erfindungsgemäß von der Koordinationseinheit bereitgestellt werden oder zumindest von der Koordinationseinheit ausgegeben werden. Die Stellung der medizinischen Diagnose kann unter Auswertung von Daten erfolgen, welche der Koordinationseinheit zur Verfügung stehen, insbesondere der Sensordaten und/oder des mindestens einen medizinischen Datensatzes. Gemäß Ausführungsformen der Erfindung gibt die Koordinationseinheit mehrere mögliche Diagnosen aus. Es ist erfindungsgemäß möglich, dass das Behandlungsschema die medizinische Diagnose mit einer oder mehreren Handlungsoptionen koppelt. Dies ermöglicht es einem Benutzer, eine Handlungsoption auszuwählen und gegebenenfalls der Koordinationseinheit eine Anweisung zu geben, dass die Handlungsoption umgesetzt werden soll.

Die Koordinationseinheit ist vorzugsweise dazu eingerichtet, eine Ausgabe des Behandlungsschemas zu veranlassen. Unter der Ausgabe ist sowohl eine graphische Ausgabe des Behandlungsschemas, zum Beispiel auf einem Bildschirm, als auch eine rein technische Ausgabe des Behandlungsschemas, beispielsweise eine Übermittlung über ein Computernetzwerk, zu verstehen. Es ist erfindungsgemäß möglich, dass die Koordinationseinheit einen Bildschirm zur Ausgabe des Behandlungsschemas aufweist. Somit kann das Behandlungsschema von einem Benutzer visuell erfasst werden. Es ist bevorzugt, wenn die Koordinationseinheit außerdem ein Eingabemittel aufweist. Dies ermöglicht es, dass die Koordinationseinheit Anfragen, Bestätigungen und sonstige Eingaben unmittelbar entgegennehmen kann. Die Koordinationseinheit bildet gemäß den vorangehenden Ausführungen also ein Werkzeug zur vereinheitlichten Kontrolle und/oder Steuerung mehrerer medizintechnischer Geräte.

Es ist bevorzugt, wenn das Eingabemittel ein Touch-Bildschirm ist. Hiermit lässt sich im klinischen Umfeld eine ausreichend komfortable Dateneingabe erzielen. Ein Touch-Bildschirm lässt sich allerdings im Vergleich zu einer Tastatur leichter reinigen. Ganz besonders bevorzugt handelt es sich bei der Koordinationseinheit um einen Tablet-Computer, also einen tragbaren Computer mit einem Touch-Bildschirm. Es können jedoch auch Computersysteme ganz anderer Art, insbesondere stationäre Computersysteme, als Koordinationseinheiten eingesetzt werden.

Gemäß einer besonderen Ausführungsform der Erfindung ist die Koordinationseinheit dazu eingerichtet, zur Auswertung der Sensordaten und/oder des mindestens einen medizinischen Datensatzes eine Anfrage an einen Berechnungsdienst zu stellen und das Behandlungsschema oder Daten, aus denen die Koordinationseinheit das Behandlungsschema ableiten kann, von dem Berechnungsdienst zu empfangen. Dies ist vor allem dann sehr nützlich, wenn die Koordinationseinheit wegen Kapazitätsgründen nicht alle zur Ableitung des Behandlungsschemas notwendigen Schritte selbststätig ausführen kann. Der Berechnungsdienst kann durch ein Kommunikationsnetz verteilter Rechner implementiert sein. Ein solches Kommunikationsnetz wird auch als eine Cloud bezeichnet. Ein Vorteil von Cloud-Lösungen besteht darin, dass eine von der Cloud zur Verfügung gestellte Rechenleistung in Abhängigkeit von der Nachfrage schnell herauf- und herunterskaliert werden kann.

Gemäß weiteren Ausführungsformen der Erfindung kann der Berechnungsdienst durch einen Großrechner oder eine Serverfarm eines Krankenhauses implementiert sein. Der Berechnungsdienst kann erfindungsgemäß Verfahren aus dem Gebiet der künstlichen Intelligenz einsetzen, so können beispielsweise Expertensysteme oder neuronale Netze zur Verwendung kommen. Auf diese Weise lassen sich aus den Sensordaten und/oder dem mindestens einen medizinischen Datensatz zeitnah Behandlungsschemata ableiten. Außerdem kann eine von der Koordinationseinheit zu tragende Rechenlast möglichst geringgehalten werden.

Es ist erfindungsgemäß möglich, dass die Koordinationseinheit dazu eingerichtet ist, eine Datenbankabfrage an eine Patientendatenbank zu stellen. Bei einer Patientendatenbank handelt es sich um ein System, das Patientendaten in Form von elektronischen Patientenakten oder sonstigen Datenbankeinträgen betreffend Patienten enthält. Es können darin beispielsweise Eckdaten wie Name, Geburtsdatum oder Wohnort eines Patienten enthalten sein, aber auch unmittelbar gesundheitsrelevante Daten wie beispielsweise Informationen zu Vorerkrankungen und aktuellen Erkrankungen, verordneten Arzneimitteln oder aktuell laufenden Behandlungsmaßnahmen. Diese Daten liegen in der Patientendatenbank vorzugsweise in einem standardisierten Format vor, das maschinell weiterverarbeitet werden kann. Medizinische Datensätze dieser Art lassen sich besonders einfach von der Koordinationseinheit nutzen. Die Koordinationseinheit kann die Patientendatenbank auch als eine Datenquelle für die Sensordaten nutzen, falls diese dort hinterlegt sind. Es ist erfindungsgemäß ferner möglich, dass die Koordinationseinheit dazu eingerichtet ist, den mindestens einen medizinischen Datensatz in einer internen Datenbank vorzuhalten oder von einer sonstigen entfernten Datenquelle abzurufen, beispielsweise aus einem Kommunikationsnetz verteilter Rechner.

Die Koordinationseinheit fragt die Sensordaten bevorzugt von den medizintechnischen Geräten ab. Dazu kann die Koordinationseinheit unmittelbar mit den medizintechnischen Geräten verbunden sein. Es ist aber auch möglich, dass eine indirekte Datenverbindung zwischen der Koordinationseinheit und den medizintechnischen Geräten besteht. So kann beispielsweise ein Kopplungsgerät vorgesehen sein, an das die medizintechnischen Geräte angeschlossen ist. Das Kopplungsgerät kommuniziert mit dem medizintechnischen Gerät vorzugsweise über eine WLAN-Verbindung.

Gemäß einem der Erfindung nicht zugehörigen Beispiel ist die Koordinationseinheit dazu eingerichtet, eine Verfügbarkeit der medizintechnischen Geräte abzufragen und bei der Veranlassung der Auswertung der Sensordaten und/oder des mindestens einen medizinischen Datensatzes zur Ableitung des Behandlungsschemas die Verfügbarkeit der medizintechnischen Geräte zu berücksichtigen. Bei der Behandlung akuter Krankheitsfälle kann es passieren, dass medizintechnische Geräte bereits belegt sind. In solchen Fällen ist es notwendig, alternative Behandlungsmöglichkeiten innerhalb kurzer Zeit aufzufinden. Ferner sollten medizinische Geräte, die tendenziell weniger stark ausgelastet sind, nach Möglichkeit genutzt werden, wenn dadurch eine Nutzung anderer, stärker angefragter medizintechnischer Geräte vermieden werden kann.

Nachfolgend wird ein hypothetisches Anwendungsbeispiel gegeben. Es wird ein Patient auf einer Intensivstation aufgenommen, der positiv auf COVID-19 getestet wurde. Bei dem Patienten liegt ein akutes Lungenversagen (ARDS) vor. Um das Ausmaß der pulmonalen Erkrankung zu erfassen, soll eine Computertomographie des Thorax durchgeführt werden. Dies erfordert es, dass der Patient über mehrere Krankenhausflure zu einem Computertomographiegerät verbracht wird. Dabei besteht ein Ansteckungsrisiko für weitere Patienten. Nach der Durchführung der Computertomographie müsste das Computertomographiegerät gereinigt werden. Das Computertomographiegerät würde somit für eine längere Zeit anderen Patienten nicht zur Verfügung stehen. Es ist zum Beispiel denkbar, dass bei einem weiteren Patienten, bei dem eigentlich dringend eine Computertomographieuntersuchung erfolgen sollte, stattdessen eine Laparotomie durchgeführt wird, weil das Computertomographiegerät noch blockiert ist. Diese Situation ist nicht zufriedenstellend. Die Koordinationseinheit stellt im Falle des COVID-19-Patienten stattdessen fest, dass auch ein Gerät zur elektrischen Impedanz-Tomographie (EIT) verfügbar ist. Dieses kann genutzt werden, um den Zustand der Lunge zu erfassen.

Die Koordinationseinheit prägt das Behandlungsschema deshalb so aus, dass eine Untersuchung des Patienten mittels des EIT-Geräts vorgeschlagen wird. EIT-Geräte messen elektrische Leitfähigkeiten innerhalb des Körpers, was Rückschlüsse auf die Beschaffenheit des Körpergewebes zulässt. Ein solches Gerät kann in gewissen Fällen alternativ zu einem Computertomographiegerät verwendet werden. Somit wird das Computertomographiegerät nicht über einen längeren Zeitraum blockiert. Bei der Ableitung des Behandlungsschemas unter Berücksichtigung der Verfügbarkeit der medizintechnischen Geräte kann die Koordinationseinheit gemäß der Erfindung nicht zugehörigen Beispielen zusätzlich ein aufgrund einer Erkrankung des Patienten bestehendes Infektionsrisiko, eine räumliche Distanz des Patienten von den medizintechnischen Geräten, eine durchschnittliche Belegungsquote der medizintechnischen Geräte und/oder weitere Parameter berücksichtigen.

Gemäß einem der Erfindung nicht zugehörigen Beispiel kann es vorgesehen sein, dass die Sensordaten und/oder der medizinische Datensatz Messwerte von einem Gerät zur elektrischen Impedanz-Tomographie, einem Gerät zur Blutgasanalyse und/oder einem Beatmungsgerät umfassen, wobei der medizinische Datensatz mindestens einen Datenwert betreffend eine Lagerung des Patienten umfasst, und wobei das Behandlungsschema aus den Sensordaten und/oder aus dem mindestens einen medizinischen Datensatz abgeleitet eine Anweisung zur Lagerung des Patienten umfasst. Gemäß diesem Beispiel wird die Koordinationseinheit unterstützend bei der Lagerungstherapie eingesetzt. Dies erfolgt vorzugsweise bei der Behandlung von Patienten, die an COVID-19 erkrankt sind und eine Beatmungstherapie über ein Beatmungsgerät erhalten. Bevorzugt werden Messwerte von einem Gerät zur elektrischen Impedanz-Tomographie, einem Gerät zur Blutgasanalyse und/oder einem Beatmungsgerät von der Koordinationseinheit ausgewertet. Weitere Messwerte können jedoch auch berücksichtigt werden. Daraus lässt sich eine geeignete Lagerungstherapie ableiten. Es können beispielsweise jedoch auch weiterem klinischem Parameter ausgewertet werden. Die Koordinationseinheit gibt Anweisungen zur Lagerung beispielsweise an das klinische Personal aus, beispielsweise über einen Bildschirm der Koordinationseinheit.

Gemäß einem der Erfindung nicht zugehörigen Beispiel umfassen die Sensordaten und/oder der medizinische Datensatz mindestens einen Gerinnungsblutwert und einen Oxygenierungswert, wobei das Behandlungsschema aus den Sensordaten und optional aus dem mindestens einen medizinischen Datensatz abgeleitet als Diagnose ein Vorliegen von Mikrothromben in einer Lunge des Patienten umfasst, und wobei das Behandlungsschema aus den Sensordaten und/oder aus dem mindestens einen medizinischen Datensatz abgeleitet eine Gabe eines Antikoagulationsmittels an den Patienten vorsieht. Gemäß diesem Beispiel ist die Koordinationseinheit so konfiguriert, dass sie ein Vorhandensein von Mikrothromben diagnostizieren kann.

Steigt beispielsweise ein Anteil von D-Dimeren im Blut stark an und verschlechtert sich die Oxygenierung, dann weist das auf eine verschlechterte Blutgerinnung hin. Das von der Koordinationseinheit generierte Behandlungsschema umfasst die Gabe des Antikoagulationsmittels zur Lösung der Mikrothromben. Es kann beispielsweise ein Behandlungsvorschlag an medizinisches Personal ergehen, dass ein Antikoagulationsmittel verabreicht werden sollte, oder die Koordinationseinheit kann die Gabe des Antikoagulationsmittels selbst auslösen, zum Beispiel durch eine Ansteuerung einer Spritzenpumpe.

Ein besonderes Anwendungsgebiet für dieses der Erfindung nicht zugehörige Beispiel ist die Behandlung von Patienten, die an COVID-19 erkrankt sind, denn nach aktuellen Erkenntnissen geht mit dieser Erkrankung eine gesteigerte Blutgerinnung einher, was zu Thrombosen und Lungenembolien führen kann. Die Koordinationseinheit kann in solchen Fällen die notwendigen vorbeugenden Schritte veranlassen. Es versteht sich, dass die Gabe des Antikoagulationsmittels auch bei sonstigen Erkrankungen von der Koordinationseinheit veranlasst werden kann, insofern dies medizinisch indiziert ist.

Gemäß einem der Erfindung nicht zugehörigen Beispiel umfassen die Sensordaten und/oder der medizinische Datensatz ferner Messwerte von einem Gerät zur elektrischen Impedanz-Tomographie, einem Gerät zur Blutgasanalyse und/oder einem Beatmungsgerät. Optional umfasst der medizinische Datensatz mindestens einen Datenwert zu einer Lagerung des Patienten. Zur Feststellung der Mikrothromben können Untersuchungsergebnisse von einem EIT-Gerät genutzt werden. Mittels EIT-Geräte ist es beispielsweise möglich, ein feinauflösendes Abbild des Thorax zu erstellen. Gemäß einem der Erfindung nicht zugehörigen Beispiel ist es möglich, dass die Koordinationseinheit dazu eingerichtet ist, ein Abbild des Thorax automatisiert auszuwerten, sodass Mikrothromben erkannt werden können. Auf dieser Grundlage kann insbesondere in Zusammenschau mit weiteren Messwerten und medizinischen Datensätzen eine Diagnose abgeleitet werden. Alternativ oder zusätzlich kann auch mindestens ein Messwert, der von einem Beatmungsgerät ausgegeben wird, ausgewertet werden, denn Atemparameter können durch das beginnende und fortschreitende Auftreten von Mikrothromben beeinflusst werden. Schließlich kann ergänzend ein Datenwert zu einer Lagerung des Patienten ausgewertet werden. Zum Beispiel kann ermittelt werden, wie häufig die Lagerungsposition verändert wurde und ob der Patient eventuell zeitweise das Krankenbett verlassen hat. Auf dieser Grundlage lässt sich eine genauere Aussage zur Wahrscheinlichkeit einer Thrombusbildung treffen.

Bei den vorangehend beschriebenen Beispielen ist es jeweils möglich, dass der medizinische Datensatz kennzeichnet, dass der Patient mit einem Erreger aus der Familie der Coronaviren infiziert ist. Dabei kann es sich insbesondere um SARS-CoV-2 handeln, das auch als schweres akutes respiratorisches Syndrom-Coronavirus-2 bezeichnet wird. Dieses ruft die Krankheit COVID-19 hervor. Die Koordinationseinheit kann beispielsweise dazu eingerichtet, dies bei der Ableitung des Behandlungsschemas zu berücksichtigen. Es versteht sich aber, dass eine solche Koordinationseinheit auch bei anderen Krankheitsfällen zum Einsatz kommen kann.

Der medizinische Datensatz verzeichnet erfindungsgemäß, dass der Patient an einer auf einer Intensivstation erworbenen Muskelschwäche leidet, wobei die Sensordaten und/oder der medizinische Datensatz Messwerte von einem Gerät zur elektrischen Impedanz-Tomographie und/oder von einem Beatmungsgerät umfasst, wobei der medizinische Datensatz optional einen Sedierungsstatus des Patienten angibt, und wobei das Behandlungsschema aus den Sensordaten und optional aus dem mindestens einen medizinischen Datensatz abgeleitet mindestens eine für einen Entwöhnungsprozess von einem Beatmungsgerät spezifische Anweisung umfasst. Unter dem Begriff der auf einer Intensivstation erworbenen Muskelschwäche, auch bekannt als ICU-Acquired Weakness, ist eine schwerwiegende Muskelerkrankung zu verstehen, die unterschiedlich ausgeprägt sein kann. Bei einer möglichen Ausprägung handelt es sich um die sogenannte Critical-Illness-Polyneuropathie, eine Erkrankung des peripheren Nervensystems. Patienten entwickeln schlaffe, atrophe Lähmungen. Dieser Erkrankung kann insbesondere infolge einer Sepsis, eines Multiorganversagens oder einer Langzeitbeatmung auftreten. Bei einer weiteren Ausprägung der Erkrankung handelt es sich um die sogenannte Critical-Illness-Myopathie, die insbesondere im Zuge pulmonaler Erkrankungen auftreten kann. Bei beiden Ausprägungen der Erkrankung kann die Entwöhnung des Patienten von einem Beatmungsgerät erheblich erschwert sein. Eine ICU-Acquired Weakness kann sich zum Beispiel an eine COVID-19-Erkrankung anschließen, wenn ein Patient auf einer Intensivstation beatmet wurde.

Die Koordinationseinheit wertet erfindungsgemäß die vorangehend genannten Datenquellen aus, um die Entwöhnung von dem Beatmungsgerät optimal zu unterstützen. Die Entwöhnung von dem Beatmungsgerät, auch als Weaning bezeichnet, ermöglicht es dem Patienten, nach einer Phase der Atemunterstützung wieder eigenständig zu atmen. Während das Weaning bei kurzen Beatmungsperioden in der Regel unproblematisch ist, kann es sich nach einer Langzeitbeatmung als schwierig darstellen. Es kann im Einzelfall mehrere Wochen dauern, bis der Patient wieder vollkommen selbstständig atmen kann. Unter assistierter Spontanatmung durch ein Beatmungsgerät wird der Patient stetig an die eigenständige Atmung herangeführt. Dabei kann eine Feinanpassung diverser Beatmungsparameter vorteilhaft sein.

Die Koordinationseinheit ist bevorzugt dazu eingerichtet, eine entsprechende Anpassung der Beatmungsparameter vorzunehmen. Besonders bevorzugt steuert die Koordinationseinheit ein Beatmungsgerät unmittelbar an. Alternativ oder zusätzlich kann die Koordinationseinheit aber auch Handlungsanweisungen an medizinisches Personal ausgeben. Daraufhin kann eine geeignete Anpassung von Beatmungsparametern manuell vorgenommen werden.

Vorzugsweise ist die für den Entwöhnungsprozess spezifische Anweisung ein Startzeitpunkt für den Entwöhnungsprozess, ein Endzeitpunkt für den Entwöhnungsprozess oder eine Anweisung zur Anpassung von Beatmungsparametern. Der Startzeitpunkt für den Entwöhnungsprozess sollte in Abhängigkeit von einem gesundheitlichen Zustand des Patienten gewählt werden. In der Regel kann mit dem Weaning erst begonnen werden, wenn Erkrankungen bereits soweit abgeklungen oder ausgeheilt sind, dass der Patient den Entwöhnungsprozess bewältigen kann. Erfindungsgemäß kann die Koordinationseinheit einen geeigneten Startzeitpunkt ermitteln. Dazu können erfindungsgemäß insbesondere folgende Datenquellen beziehungsweise Parameter von der Koordinationseinheit verwendet werden: Herzfrequenz, Blutdruck, Entzündungsparameter (z.B. CRP-Wert), Körpertemperatur, Sedierungstiefe, Blutgasanalysewerte (z.B. pO2-Wert, pCO2-Wert, pH-Wert, Hämoglobinspiegel), Ausgabedaten bildgebender Verfahren (z.B. eines MRT-Geräts oder eines EIT-Geräts), Bedarf an kreislaufunterstützenden Medikamenten (z.B. abgerufen aus Konfigurationsdaten einer Dauerspritzenpumpe) und/oder Flüssigkeitsbilanz (z.B. Zufuhr an Infusionen, Enteralia und Medikamenten im Verhältnis zu Ausscheidungen oder Flüssigkeitsbilanz eines Dialysegerätes). Weitere Datenquellen beziehungsweise Parameter können erfindungsgemäß jedoch ebenfalls zur Ermittlung des Startzeitpunkts herangezogen werden.

Gleichwohl kann die Koordinationseinheit den Endzeitpunkt für den Entwöhnungsprozess vorschlagen. Dabei berücksichtigt die Koordinationseinheit bevorzugt insbesondere folgende Datenquellen beziehungsweise Parameter: Atemarbeit (z.B. ermittelt durch ein Beatmungsgerät), RSBI (Rapid Shallow Breathing Index, Verhältnis der Atemfrequenz zum Tidalvolumen), Herzfrequenz, Blutdruck, Entzündungsparameter (z.B. CRP-Wert), Körpertemperatur, Sedierungstiefe, Blutgasanalysewerte (z.B. pO2-Wert, pCO2-Wert, pH-Wert, Hämoglobinspiegel), Ausgabedaten bildgebender Verfahren (z.B. eines MRT-Geräts oder eines EIT-Geräts) und/oder Flüssigkeitsbilanz (z.B. abgerufen aus Konfigurationsdaten einer Dauerspritzenpumpe). Weitere Datenquellen beziehungsweise Parameter können erfindungsgemäß jedoch ebenfalls zur Ermittlung des Endzeitpunkts herangezogen werden.

Bei der Anweisung zur Anpassung von Beatmungsparametern kann es sich erfindungsgemäß um eine Anweisung handeln, die darauf gerichtet ist, eine Fehlanpassung des Beatmungsgeräts an den Patienten zu verhindern. So kann erfindungsgemäß ermittelt werden, ob eine Ösophagus-Drucknegativierung ohne Druckabgabe durch das Beatmungsgerat bei Spontanatmenden vorliegt. Dies weist auf eine sogenannte Triggerasynchronität hin. Dies heißt, dass das Beatmungsgerät unter Spontanatmung bei Einatmungsvorgängen nicht anspricht und den Patienten somit nicht bei der Atmung unterstützt. Erfindungsgemäß kann ferner ermittelt werden, ob eine Ösophagus-Drucknegativierung vor der Druckabgabe durch das Beatmungsgerät bei spontanatmenden Patienten vorliegt. Dies kann auf eine sogenannte Autotriggerung hinweisen, das heißt, dass das Beatmungsgerät einsetzt, obwohl der Patient noch gar nicht mit der Einatmung begonnen hat. Beide genannten Fälle können den Entwöhnungsprozess behindern. Dementsprechend kann die Koordinationseinheit ein Beatmungsgerät korrigierend ansteuern und/oder einen Handlungsvorschlag zur Umsetzung durch medizinisches Personal ausgeben. Somit können Korrekturen vorgenommen werden, die einen Erfolg des Weanings begünstigen.

Es ist vorteilhaft, wenn das Behandlungsschema eine Anweisung zur Einstellung einer Beatmungsfrequenz, zur Einstellung einer Höhe eines Beatmungsdrucks und/oder zur Einstellung eines Tidalvolumens zur Ermöglichung einer Spontanatmung umfasst. Diese Parameter sollten bevorzugt in Abhängigkeit von dem Verlauf des Weanings laufend angepasst werden. Es ist besonders vorteilhaft, wenn die Koordinationseinheit das Behandlungsschema so ausprägt, dass eine zwerchfellprotektive Beatmung erfolgt. Bei einer maschinellen Beatmung besteht die Möglichkeit von Lungenschädigungen. Das Risiko für eine Lungenschädigung kann allerdings durch eine geeignete Anpassung diverser Beatmungsparameter verringert werden. So sollte zum Beispiel der Beatmungsdruck, also der maximale inspiratorische Druck, möglichst gering gehalten werden. Bei der Einstellung des Tidalvolumens ist zu berücksichtigen, dass das Tidalvolumen umso kleiner gewählt werden sollte, je geringer die sogenannte Compliance (Dehnungsfähigkeit) der Lunge ist. Eine zwerchfellschonende Beatmung beim Weaning kann unter Berücksichtigung dieser und optional weiterer Parameter mittels der erfindungsgemäßen Koordinationseinheit erfolgen.

Gemäß einem der Erfindung nicht zugehörigen Beispiel stammen die Sensordaten von einem Gerät zur elektrischen Impedanz-Tomographie, einem Beatmungsgerät, einem Gerät zur Pulsoxymetrie, einem Hämoglobin-Messgerät, einem Ösophagusdruck-Sensor, einem Gerät zur Blutdruckmessung, einem Computertomographen, einem Röntgengerät, einem Ultraschallgerät, einem Kapnometer, einem EEG-Gerät und/oder einer Vorrichtung zur Blutgasanalyse, und das Behandlungsschema umfasst mindestens eine zur Behandlung einer akuten respiratorischen Insuffizienz des Patienten spezifische Anweisung, die aus den Sensordaten und/oder dem medizinischen Datensatz abgeleitet ist. Gemäß dieser Ausführungsform wird eine Behandlung einer akuten respiratorischen Insuffizienz eines Patienten eingeleitet, bei der die obengenannten Sensordaten und/oder Messwerte miteinbezogen werden, um eine bestmögliche Behandlung zu gewährleisten.

Die mindestens eine zur Behandlung der akuten respiratorischen Insuffizienz des Patienten spezifische Anweisung ist gemäß einem der Erfindung nicht zugehörigen Beispiel eine Anweisung zur Einstellung einer inspiratorischen O2-Konzentration, eine Anweisung zur Einstellung einer Inspirationszeit, eine Anweisung zur Einstellung eines PEEP-Wertes, eine Anweisung zur Einstellung einer mandatorischen Beatmungsfrequenz, eine Anweisung zur Einstellung eines individuellen Triggers und/oder eine Anweisung zur Einstellung eines Sedierungsparameters. In vielen Fällen kann es vorteilhaft sein, wenn die Koordinationseinheit die Behandlung der akuten respiratorischen Insuffizienz unterstützt. Nachfolgend wird ein Beispielfall angegeben. Auf einer Intensivstation wird ein beatmungspflichtiger Patient aufgenommen, bei dem ein Verdacht auf eine COVID-19-Erkrankung besteht. Es liegen ein ausgeprägter Atemantrieb, ein hoher Sedierungsbedarf und eine nahezu normale Compliance der Lunge vor. Eine bildgebende Diagnostik unter Einsatz beispielsweise eines MRT-Geräts ist aufgrund der instabilen pulmonalen Situation risikobehaftet. Eine lungenprotektive Beatmung zur Vermeidung von biomechanischen Komplikationen aufgrund einer endinspiratorischen Überdehnung gestaltet sich sehr schwierig. Durch die fortlaufende Bewertung der Lungensituation, dem notwendigen Sedierungsregime, der Blutgase und der elektrischen Impedanztomographie kann ein sicheres Beatmungsfenster mit reduzierter endinspiratorischer Überdehnung durch Einstellung eines individuellen PEEP-Wertes erreicht werden. Das Kürzel PEEP bezeichnet den positiven endexspiratorischen Druck bei der Atmung. Es sollte bei der künstlichen Überdruckbeatmung beim Ende der Ausatmung ein positiver Druck bestehen, um einen Kollaps der Lungenbläschen zu vermeiden. Dazu kann ein sogenanntes PEEP-Ventil verwendet werden.

Bei sämtlichen vorangehend beschriebenen und der Erfindung zugehörigen Behandlungsvorgängen, die Lungenerkrankungen betreffen, kann die Koordinationseinheit erfindungsgemäß dazu eingerichtet sein, aus diversen Messwerten Handlungsanweisungen abzuleiten, die in das Behandlungsschema einfließen. Das entsprechende Behandlungswissen, das gemäß Ausführungsformen von der Koordinationseinheit angewendet werden kann, ist nachfolgend aufgeführt. Gemäß einer vorteilhaften Ausführungsform der Erfindung ist die Koordinationseinheit dazu eingerichtet, in einer Phase einer akuten respiratorischen Insuffizienz des Patienten Parameter umfassend einen Blutdruck, einen SpO2-Wert (Sauerstoffsättigung des Bluts, beispielsweise gemessen mittels Pulsoxymetrie), einen Hämoglobinspiegel und/oder einen pO2-Wert zu berücksichtigen. Daraus kann die Koordinationseinheit erfindungsgemäß eine notwendige inspiratorischen O2-Konzentration und/oder eine günstige Inspirationszeit ableiten. Gemäß einer weiteren vorteilhaften Ausführungsform der Erfindung ist die Koordinationseinheit dazu eingerichtet, in einer Phase einer akuten respiratorischen Insuffizienz des Patienten transpulmonale Drücke am Ende der Ausatmung und/oder am Ende der Einatmung zu messen und daraus eine individuelle Einstellung eines PEEP-Wertes für ein Beatmungsgerät abzuleiten. Gemäß einer weiteren vorteilhaften Ausführungsform der Erfindung ist die Koordinationseinheit dazu eingerichtet, in einer Phase einer akuten respiratorischen Insuffizienz des Patienten Ausgabedaten eines Computertomographiegeräts, eines Röntgengeräts und/oder eines Ultraschallgeräts als Ausgangsbasis für eine elektrische Impedanztomographie zu berücksichtigen und daraus eine Diagnose einer Lungensituation des Patienten abzuleiten. Die Diagnose kann zur Folge haben, dass eine Intensivierung der Spontanatmung durch die Koordinationseinheit veranlasst wird.

Gemäß einer weiteren vorteilhaften Ausführungsform der Erfindung ist die Koordinationseinheit dazu eingerichtet, in einer Phase einer akuten respiratorischen Insuffizienz des Patienten einen PEEP-Wert, einen pCO2-Wert, einen Inspirationsdruck, ein Tidalvolumen und/oder ein Ergebnis einer volumetrischen Kapnometrie zu berücksichtigen, um eine mandatorische Beatmungsfrequenz eines Beatmungsgeräts einzustellen. Gemäß einer weiteren vorteilhaften Ausführungsform der Erfindung ist die Koordinationseinheit dazu eingerichtet, in einer Phase einer akuten respiratorischen Insuffizienz des Patienten eine Flowkurve und/oder eine Ösophaguskurve auszuwerten, um einen Triggerparameter zur Auslösung einer Beatmung durch ein Beatmungsgerät einzustellen. Die Flowkurve zeigt ein Atemvolumen über die Zeit an. Die Ösophaguskurve zeigt einen Ösophagusdruck über die Zeit an. Durch die genaue Einstellung des Triggerparameters kann es verhindert werden, dass die Atemunterstützung nicht ausgelöst wird oder dass das Beatmungsgerät einen Beatmungsvorgang auslöst, obwohl dies nicht angezeigt ist.

Gemäß einer weiteren vorteilhaften Ausführungsform der Erfindung ist die Koordinationseinheit dazu eingerichtet, in einer Phase einer akuten respiratorischen Insuffizienz des Patienten eine Einstellung einer Dauerspritzenpumpe, ein Atemmuster, eine Atemfrequenz, eine Herzfrequenz, einen Blutdruck, eine Ösophaguskurve und/oder EEG-Messwerte auszuwerten, um eine notwendige Sedierungstiefe abzuleiten. Daraus können von der Koordinationseinheit Handlungsanweisungen an medizinisches Personal zur Gabe notwendiger Medikamente für die Sedierung abgeleitet werden. Gemäß einer weiteren vorteilhaften Ausführungsform der Erfindung ist die Koordinationseinheit dazu eingerichtet, in einer Phase einer akuten respiratorischen Insuffizienz des Patienten eine Einstellung einer Dauerspritzenpumpe für volatile Anästhetika, ein Atemmuster, eine Atemfrequenz, eine Herzfrequenz, einen Blutdruck, eine Ösophaguskurve, EEG-Messwerte und/oder Ergebnisse einer endtidalen Narkosegasmessung auszuwerten, um eine notwendige Sedierungstiefe abzuleiten. Daraus können von der Koordinationseinheit Parameter zur Einstellung von Narkosegasen, die für die Sedierung notwendig sind, abgeleitet werden.

Gemäß einer weiteren vorteilhaften Ausführungsform der Erfindung ist die Koordinationseinheit dazu eingerichtet, in einer Stabilisierungsphase nach einer akuten respiratorischen Insuffizienz eines Patienten ein Verhältnis einer Atemfrequenz zu einem Tidalvolumen, einen pO2-Wert und/oder einen pCO2-Wert auszuwerten, um eine Anpassung einer Frequenz der maschinellen Atemzüge, eine Anpassung einer Höhe eines Beatmungsdrucks bei einer maschinellen Beatmung und/oder eine Anpassung eines Beatmungsdrucks bei einer maschinellen Beatmung zu veranlassen. Unter der Stabilisierungsphase soll ein Zeitraum nach einer akuten respiratorischen Insuffizienz eines Patienten verstanden werden, in dem noch nicht mit dem Weaning begonnen wurde. Gemäß einer weiteren vorteilhaften Ausführungsform der Erfindung ist die Koordinationseinheit dazu eingerichtet, in einer Stabilisierungsphase nach einer akuten respiratorischen Insuffizienz eines Patienten ein Druckdelta zwischen einem PEEP-Wert und einem geringsten Ösophagusdruck bei der Inspiration zu ermitteln, um ein Maß zu ermitteln, das widergibt, ob eine inadäquate Spontanatmung vorliegt. Dieses Maß kann von der Koordinationseinheit erfindungsgemäß als eine Diagnose ausgegeben werden.

Gemäß einem zweiten Aspekt der Erfindung wird ein Behandlungssystem vorgeschlagen. Das Behandlungssystem umfasst die vorangehend beschriebene Koordinationseinheit und mindestens ein medizintechnisches Gerät, wobei die Koordinationseinheit mit dem medizintechnischen Gerät über eine Datenverbindung gekoppelt ist. Die Datenverbindung kann dabei grundsätzlich beliebig ausgeprägt sein, bevorzugt sollte es sich dabei aber um eine drahtlose Datenverbindung wie eine WLAN-Verbindung handeln. Somit kann das Behandlungssystem Sensordaten der medizintechnischen Geräte empfangen und Anweisungen zur Umsetzung des Behandlungsschemas an die medizintechnischen Geräte ausgeben. Eine Kopplung der Koordinationseinheit mit den medizintechnischen Geräten über ein drahtgebundenes Netzwerk ist ebenfalls möglich. Es ist auch möglich, dass die Datenverbindung über mehrere Zwischenstationen aufgebaut wird.

Das Behandlungssystem weist bevorzugt einen Berechnungsdienst auf, wobei die Koordinationseinheit mit dem Berechnungsdienst über eine Datenverbindung gekoppelt ist. Der Berechnungsdienst kann erfindungsgemäß Verfahren aus dem Gebiet der künstlichen Intelligenz einsetzen. So kann der Berechnungsdienst erfindungsgemäß so konfiguriert sein, dass er ein Expertensystem ausführt. Der Berechnungsdienst kann ferner dazu eingerichtet sein, ein neuronales Netz zu nutzen, um aus den Sensordaten und/oder dem mindestens einen medizinischen Datensatz Behandlungsschemata und/oder medizinische Diagnosen abzuleiten. Der Berechnungsdienst kann ferner dazu eingerichtet sein, bei der Ableitung des Behandlungsschemas vergleichbare Krankheitsverläufe anderer Patienten zu berücksichtigen, welche er aus einer Datenbank abruft. Die von der Koordinationseinheit zu tragende Rechenlast kann durch die Auslagerung auf den Berechnungsdienst geringgehalten werden. Der Berechnungsdienst wird bevorzugt durch in einem Kommunikationsnetz verteilte Rechner bereitgestellt, also ein Cloud-System. Gemäß anderen Ausführungsformen der Erfindung kann der Berechnungsdienst durch einen Server oder eine Serverfarm eines Krankenhauses implementiert sein, in dem die Koordinationseinheit zum Einsatz kommt.

Es ist erfindungsgemäß ferner möglich, dass das Behandlungssystem einen Unterstützungsrechner aufweist, der mit der Koordinationseinheit über eine Datenverbindung gekoppelt ist, wobei der Unterstützungsrechner zur Ausführung von Berechnungen für die Koordinationseinheit und/oder zur Bereitstellung von medizinischen Datensätzen und/oder zur Bereitstellung von Sensordaten an die Koordinationseinheit eingerichtet ist. Der Einsatz eines solchen Unterstützungsrechners ist vor allem dann sinnvoll, wenn die Koordinationseinheit ein tragbares Gerät mit eingeschränkter Rechenleistung ist, zum Beispiel ein Tablet-Computer. Wichtige Programme und Daten können in diesem Fall von dem Unterstützungsrechner bereitgehalten werden und bei Bedarf an den Tablet-Computer übermittelt werden. Der Unterstützungsrechner kann erfindungsgemäß der Durchführung umfangreicher Berechnungen übernehmen. Bei dem Unterstützungsrechner kann es sich beispielsweise um einen Server eines Krankenhauses handeln. Das Behandlungssystem kann erfindungsgemäß so ausgebildet sein, dass es eine Mehrzahl von Koordinationseinheiten umfasst, die den Unterstützungsrechner nutzen.

Der Unterstützungsrechner weist bevorzugt eine Datenbank auf. Gemäß einer besonders vorteilhaften Variante der Erfindung handelt es sich bei der Datenbank um eine Anästhesie-Datenbank. Die Anästhesie-Datenbank verzeichnet Informationen, die von Anästhesiegeräten und optional weiteren medizintechnischen Geräten aufgenommen werden. Der Unterstützungsrechner kann somit als ein Anästhesieserver dienen. In der Datenbank können aber auch sonstige Patientendaten beziehungsweise Sensordaten (z.B. Dosierungsvorgaben für Medikamente in Abhängigkeit der Leber- und Nierensituation) archiviert werden. Ferner können darin Daten zur Verfügbarkeit von medizintechnischen Geräten gespeichert sein. Dies ermöglicht es, bei der Erstellung von Behandlungsschemata die Verfügbarkeit der medizintechnischen Geräte mit einzubeziehen.

Es ist erfindungsgemäß ferner möglich, dass der Unterstützungsrechner dazu eingerichtet ist, Informationen von einem Krankenhaus-Informationssystem über eine Datenverbindung abzufragen und die Informationen der Koordinationseinheit bereitzustellen, wobei die Informationen die Sensordaten und/oder die medizinischen Datensätze umfassen. Bei einem Krankenhaus-Informationssystem handelt es sich um ein EDV-System, das unterschiedlichste Aufgaben aus Bereichen wie Administration, Dokumentation und/oder Koordination innerhalb eines Krankenhauses unterstützt. In dem Krankenhaus-Informationssystem können beispielsweise elektronische Patientenakten geführt werden. Gemäß vorteilhaften Ausführungsformen der Erfindung ist es möglich, dass die Koordinationseinheit mittels des Unterstützungsrechners den Inhalt von Patientenakten oder sonstige medizinische Datensätze aus dem Krankenhaus-Informationssystem abruft. Diverse medizintechnische Geräte können mit dem Krankenhaus-Informationssystem verbunden sein und Sensordaten an dieses liefern. Es ist folglich möglich, dass die Koordinationseinheit mittels des Unterstützungsrechners Sensordaten von dem Krankenhaus-Informationssystem abfragt. Das Krankenhaus-Informationssystem kann auf einem oder mehreren Rechnern des Krankenhauses implementiert sein, es kann aber zumindest teilweise auch auf einem zentralen Server des Krankenhauses oder in einem Cloud-System ausgeführt werden.

In den Zeichnungen wird eine vorteilhafte Ausführungsform der Erfindung beispielhaft erläutert. Dabei zeigt:
Fig. 1 eine schematische Darstellung eines Behandlungssystems und
Fig. 2 ein Ablaufdiagramm mit Schritten, welche von einer Koordinationseinheit zur Ableitung eines Behandlungsschemas ausgeführt werden.

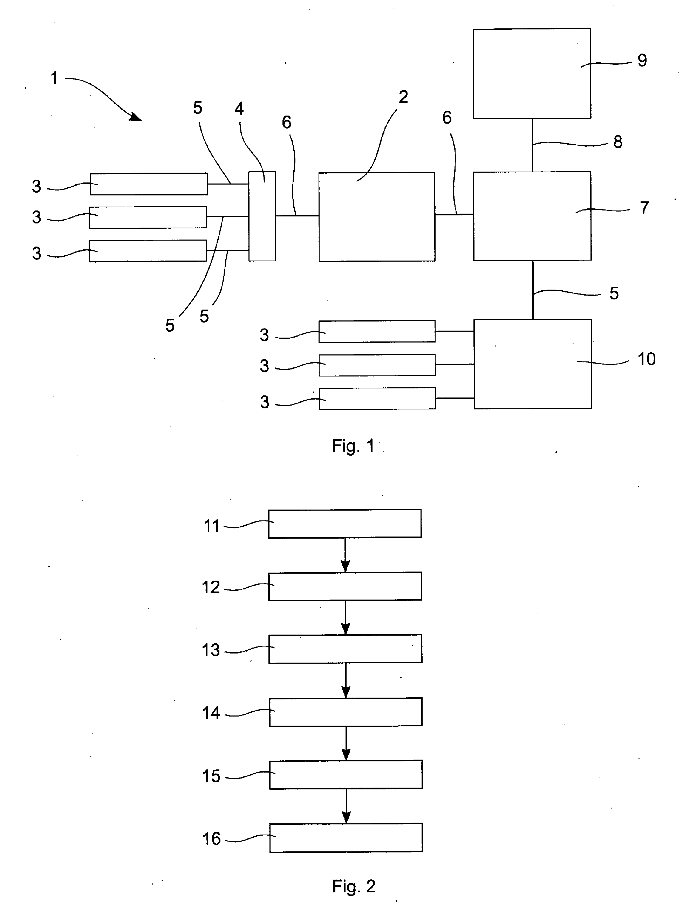

Fig. 1 zeigt eine schematische Darstellung eines Behandlungssystems 1. Das Behandlungssystem 1 weist eine Koordinationseinheit 2 auf. Die Koordinationseinheit 2 ist mit mehreren medizintechnischen Geräten 3 über eine Koppeleinheit 4 verbunden. Die Koppeleinheit 4 ist über drahtgebundene Datenverbindungen 5 mit den medizintechnischen Geräten 3 verbunden. Die Koordinationseinheit 2 ist über eine Funkverbindung 6, bei der es sich um eine WLAN-Verbindung handelt, mit der Koppeleinheit 4 verbunden. Somit kann die Koordinationseinheit 2 Sensordaten von den medizintechnischen Geräten 3 mittels der Koppeleinheit 4 erhalten und die medizintechnischen Geräte 3 auch ansteuern.

Die Koordinationseinheit 2 ist über eine weitere Funkverbindung 6 mit einem Unterstützungsrechner 7 verbunden. Der Unterstützungsrechner 7 dient als ein Anästhesieserver und erlaubt die Dokumentation von Behandlungsschritten bei der Anästhesie. Darüber hinaus kann der Unterstützungsrechner 7 aber auch weitere Behandlungsdaten speichern. Ferner stellt er der Koordinationseinheit 2 Behandlungsdaten und Programme bereit. Aufwändige Berechnungen, die nicht durch die Koordinationseinheit 2 ausgeführt werden können, kann der Unterstützungsrechner 7 übernehmen. Der Unterstützungsrechner 7 ist über eine Internetverbindung 8 mit einem Berechnungsdienst 9 verbunden. Die Koordinationseinheit 2 kann somit über den Unterstützungsrechner 7 eine Anfrage an den Berechnungsdienst 9 stellen. Die Anfrage kann beispielsweise Sensordaten und einen medizinischen Datensatz wie einen Auszug aus einer Patientenakte enthalten. Der Berechnungsdienst 9 wird durch vernetzte Rechner in einem Cloud-System implementiert. Der Berechnungsdienst 9 stellt Dienste unter Nutzung von Verfahren aus dem Bereich der künstlichen Intelligenz bereit. Somit kann er aus den Sensordaten und weiteren medizinischen Daten Behandlungsschemata ableiten, die medizinische Diagnosen und Handlungsempfehlungen enthalten.

Der Unterstützungsrechner 7 ist ferner mit einem Krankenhaus-Informationssystem 10 über eine weitere drahtgebundene Datenverbindung 5 gekoppelt. Das Krankenhaus-Informationssystem 10 unterstützt Aufgaben aus den Bereichen Administration, Dokumentation und Koordination und wird durch eine Vielzahl von Rechnern gebildet. Aus dem Krankenhaus-Informationssystem 10 kann die Koordinationseinheit 2 Patientendaten abfragen. Da zumindest einige Rechner des Krankenhaus-Informationssystems 10 auch mit medizintechnischen Geräten 3 verbunden sind, können darüber auch Sensorwerte der medizintechnischen Geräte 3 abgerufen werden.

Fig. 2 zeigt ein Ablaufdiagramm mit Schritten, welche von der Koordinationseinheit zur Ableitung eines Behandlungsschemas ausgeführt werden. In einem ersten Empfangsschritt 11 empfängt die Koordinationseinheit Sensordaten. Die Sensordaten stammen von medizintechnischen Geräten, die direkt oder indirekt mit der Koordinationseinheit verbunden sind. In einem zweiten Empfangsschritt 12 empfängt die Koordinationseinheit einen medizinischen Datensatz. Der medizinische Datensatz kann beispielsweise aus einer elektronischen Patientenakte stammen. In einem nachfolgenden Auswertungsschritt 13 wertet die Koordinationseinheit die Sensordaten und den medizinischen Datensatz aus. Dazu notwendige Rechenschritte können unmittelbar von der Koordinationseinheit ausgeführt werden. In Abhängigkeit von der bearbeiteten Aufgabenstellung können Rechenschritte jedoch auch von dem Unterstützungsrechner oder von dem Berechnungsdienst übernommen werden. Das Ergebnis des Auswertungsschritts 13 ist ein Behandlungsschema. Das Behandlungsschema enthält Schritte, die zur medizinischen Behandlung eines Patienten notwendig sind. In einem ersten Anweisungsschritt 14 steuert die Koordinationseinheit mindestens ein medizintechnisches Gerät so an, dass das Behandlungsschema umgesetzt wird. In einem zweiten Anweisungsschritt 15 gibt die Koordinationseinheit das Behandlungsschema auf einem Bildschirm der Koordinationseinheit aus, wobei in dem Behandlungsschema Anweisungen an medizinisches Personal enthalten sind. Somit kann die Koordinationseinheit auch Behandlungsschritte veranlassen, die nicht durch die medizintechnischen Geräte umgesetzt werden können. In einem Diagnoseschritt 16 gibt die Koordinationseinheit schließlich eine medizinische Diagnose aus, die aus den Sensordaten und dem medizinischen Datensatz abgeleitet worden ist. Die Koordinationseinheit unterstützt somit das medizinische Fachpersonal bei der Diagnose von Krankheiten und kann die notwendigen Schritte zur Behandlung von Patienten veranlassen.

### Bezugszeichenliste

1. Behandlungssystem
2. Koordinationseinheit
3. Medizintechnisches Gerät
4. Koppeleinheit
5. Drahtgebundene Datenverbindung
6. Funkverbindung
7. Unterstützungsrechner
8. Internetverbindung
9. Berechnungsdienst
10. Krankenhaus-Informationssystem
11.Erster Empfangsschritt
12.Zweiter Empfangsschritt
13.Auswertungsschritt
14.Erster Anweisungsschritt
15.Zweiter Anweisungsschritt
16. Diagnoseschritt

## Patentansprüche

1. Koordinationseinheit (2) für eine Gruppe von medizintechnischen Geräten (3), wobei die Koordinationseinheit (2) dazu eingerichtet ist, die folgenden Schritte auszuführen:
- Empfang von Sensordaten von mindestens einem der medizintechnischen Geräte (3), wobei die Sensordaten einem Patienten zugeordnet sind,
- Empfang von einem medizinischen Datensatz, der dem Patienten zugeordnet ist, und
- Veranlassung einer Auswertung der Sensordaten und/oder des mindestens einen medizinischen Datensatzes, um ein Behandlungsschema abzuleiten,
**dadurch gekennzeichnet, dass**
der medizinische Datensatz verzeichnet, dass der Patient an einer auf einer Intensivstation erworbenen Muskelschwäche leidet, wobei die Sensordaten und/oder der medizinische Datensatz Messwerte von einem Gerät zur elektrischen Impedanz-Tomographie und/oder von einem Beatmungsgerät umfasst, wobei der medizinische Datensatz optional einen Sedierungsstatus des Patienten angibt, und wobei das Behandlungsschema aus den Sensordaten und optional aus dem mindestens einen medizinischen Datensatz abgeleitet mindestens eine für einen Entwöhnungsprozess von einem Beatmungsgerät spezifische Anweisung umfasst.

2. Koordinationseinheit (2) nach Anspruch 1, **dadurch gekennzeichnet, dass** die Koordinationseinheit (2) ferner dazu eingerichtet ist, mindestens eines der medizintechnischen Geräte (3) so anzusteuern, dass eine Umsetzung des Behandlungsschemas erfolgt und/oder die Umsetzung des Behandlungsschemas unterstützt wird.

3. Koordinationseinheit (2) nach Anspruch 2, **dadurch gekennzeichnet, dass** die Koordinationseinheit (2) dazu eingerichtet ist, das Behandlungsschema auszugeben, ein Bestätigungssignal zu erhalten, und nur bei Erhalt des Bestätigungssignals das mindestens eine der medizintechnischen Geräte (3) so anzusteuern, dass die Umsetzung des Behandlungsschemas erfolgt oder die Umsetzung des Behandlungsschemas unterstützt wird, wobei das Behandlungsschema eine medizinische Diagnose beinhaltet.

4. Koordinationseinheit (2) nach einem der vorhergehenden Ansprüche,
**dadurch gekennzeichnet, dass** die Koordinationseinheit (2) dazu eingerichtet ist, eine Ausgabe des Behandlungsschemas zu veranlassen, wobei die Koordinationseinheit (2) einen Bildschirm zur Ausgabe des Behandlungsschemas aufweist.

5. Koordinationseinheit (2) nach einem der vorhergehenden Ansprüche,
**dadurch gekennzeichnet, dass** die Koordinationseinheit (2) ein Eingabemittel aufweist, wobei das Eingabemittel ein Touch-Bildschirm ist.

6. Koordinationseinheit (2) nach einem der vorhergehenden Ansprüche,
**dadurch gekennzeichnet, dass** die Koordinationseinheit (2) dazu eingerichtet ist, zur Auswertung der Sensordaten und/oder des mindestens einen medizinischen Datensatzes eine Anfrage an einen Berechnungsdienst (9) zu stellen und das Behandlungsschema oder Daten, aus denen die Koordinationseinheit (2) das Behandlungsschema ableiten kann, von dem Berechnungsdienst (9) zu empfangen und dass die Koordinationseinheit (2) dazu eingerichtet ist, eine Datenbankabfrage an eine Patientendatenbank zu stellen.

7. Koordinationseinheit (2) nach einem der vorhergehenden Ansprüche,
**dadurch gekennzeichnet, dass** die mindestens eine für den Entwöhnungsprozess spezifische Anweisung einen Startzeitpunkt für den Entwöhnungsprozess, einen Endzeitpunkt für den Entwöhnungsprozess oder eine Anweisung zur Anpassung eines Beatmungsparameters ist.

8. Koordinationseinheit (2) nach einem der vorhergehenden Ansprüche,
**dadurch gekennzeichnet, dass** das Behandlungsschema eine Anweisung zur Einstellung einer Beatmungsfrequenz, zur Einstellung einer Höhe eines Beatmungsdrucks und/oder zur Einstellung eines Tidalvolumens zur Ermöglichung einer Spontanatmung umfasst.

9. Behandlungssystem (1) umfassend eine Koordinationseinheit (2) und mindestens ein medizintechnisches Gerät (3), wobei die Koordinationseinheit (2) mit dem medizintechnischen Gerät (3) über eine Datenverbindung gekoppelt ist,
**dadurch gekennzeichnet, dass**
die Koordinationseinheit (2) nach einem der Ansprüche 1 bis 8 ausgebildet ist.

10. Behandlungssystem (1) nach Anspruch 9, **dadurch gekennzeichnet, dass** das Behandlungssystem (1) einen Berechnungsdienst (9) aufweist, wobei die Koordinationseinheit (2) mit dem Berechnungsdienst (9) über eine Datenverbindung gekoppelt ist, wobei der Berechnungsdienst (9) durch in einem Kommunikationsnetz verteilte Rechner gebildet wird.

11. Behandlungssystem (1) nach einem der Ansprüche 9 oder 10, **dadurch gekennzeichnet, dass** das Behandlungssystem einen Unterstützungsrechner (7) aufweist, der mit der Koordinationseinheit (2) über eine Datenverbindung gekoppelt ist, wobei der Unterstützungsrechner (7) zur Ausführung von Berechnungen für die Koordinationseinheit (2) und/oder zur Bereitstellung von medizinischen Datensätzen und/oder zur Bereitstellung von Sensordaten an die Koordinationseinheit (2) eingerichtet ist und der Unterstützungsrechner (7) eine Datenbank aufweist.

12. Behandlungssystem (1) nach Anspruch 11, **dadurch gekennzeichnet, dass** der Unterstützungsrechner (7) dazu eingerichtet ist, Informationen von einem Krankenhaus-Informationssystem (10) über eine Datenverbindung abzufragen und die Informationen der Koordinationseinheit (2) bereitzustellen, wobei die Informationen die Sensordaten und/oder die medizinischen Datensätze umfassen.

## Claims

1. A coordination unit (2) for a group of medical devices (3), wherein the coordination unit (2) is configured to execute the following steps:
- receiving sensor data from at least one of the medical devices (3), wherein the sensor data are assigned to a patient,
- receiving a medical data set that is assigned to the patient, and
- instigating an assessment of the sensor data and/or the at least a medical data set in order to derive a treatment regimen, **characterized in that** the medical data set records that the patient suffers from muscle weakness acquired in an intensive care unit, wherein the sensor data and/or the medical data set comprises measured values from a device for electrical impedance tomography and/or from a ventilator, wherein the medical set optionally indicates a sedation status of the patient, and wherein the treatment regimen derived from the sensor data and optionally from the at least one medical data set comprises at least specific instruction for a process to wean from a ventilator.

2. The coordination unit (2) according to claim 1, **characterized in that** the coordination unit (2) is furthermore configured to control at least one of the medical devices (3) such that the treatment regimen is implemented, and/or the implementation of the treatment regiment is supported.

3. The coordination unit (2) according to claim 2, **characterized in that** the coordinating unit (2) is configured to control the treatment scheme to output a confirmation signal and, only upon receipt of the confirmation signal, to control the at least one of the medical devices (3) such that the treatment regimen is implemented or the implementation or the treatment regimen is supported, wherein the treatment regimen includes a medical diagnosis.

4. The coordination unit (2) according to one of the preceding claims, **characterized in that** the coordination unit (2) is configured to initiate an output of the treatment regimen, wherein the coordination unit (2) has a screen to output the treatment regimen.

5. The coordination unit (2) according to one of the preceding claims, **characterized in that** the coordination unit (2) has an input means, wherein the input means is a touch screen.

6. The coordination unit (2) according to one of the preceding claims, **characterized in that** the coordination unit (2) is configured to submit a query to a computation service (9) to assess the sensor data and/or the at least one medical data set, and to receive from the computation service (9) the treatment regimen or data from which the coordination unit (2) can derive the treatment regimen, and that the coordination unit (2) is configured to submit a database query to a patient database.

7. The coordination unit (2) according to one of the preceding claims, **characterized in that** the at least one specific instruction for the weaning process is a start time for the weaning process, an end time for the weaning process, or an instruction for adapting a ventilation parameter.

8. The coordination unit (2) according to one of the preceding claims, **characterized in that** the treatment regimen comprises an instruction for setting a ventilation frequency, for setting a level of a ventilation pressure, and/or for setting a tidal volume to enable spontaneous breathing.

9. A treatment system (1) comprising a coordination unit (2) and at least one medical device (3), wherein the coordination unit (2) is coupled to the medical device (3) via a data link, **characterized in that** the coordination unit (2) is designed according to one of claims 1 to 8.

10. The treatment system (1) according to claim 9, **characterized in that** the treatment system (1) has a computation service (9), wherein the coordination unit (2) is coupled to the computation service (9) via a data link, wherein the computation service (9) is formed by computers distributed in a communication network.

11. The treatment system (1) according to one of claims 9 or 10, **characterized in that** the treatment system has a support computer (7) which is coupled to the coordination unit (2) via a data link, wherein the support computer (7) is configured to execute calculations for the coordination unit (2), and/or to provide medical data sets, and/or to provide sensor data to the coordination unit (2), and the support computer (7) has a database.

12. The treatment system (1) according to claim 11, **characterized in that** the support computer (7) is configured to query information from a hospital information system (10) via a data link and to provide the information to the coordination unit (2), wherein the information comprises the sensor data and/or the medical data sets.

## Revendications

1. Unité de coordination (2) pour un groupe d'appareils techniques médicaux (3), l'unité de coordination (2) étant agencée pour effectuer les étapes suivantes :
- réception de données de capteurs d'au moins un des appareils techniques médicaux (3), les données des capteurs étant attribuées à un patient,
- réception d'un jeu de données médicales, lequel est attribué au patient, et
- déclenchement d'une évaluation des données de capteur et/ou de l'au moins un jeu de données médicales afin de dériver un schéma de traitement,
**caractérisé en ce que** le jeu de données médicales enregistre que le patient souffre d'une faiblesse musculaire acquise dans une unité de soins intensifs, les données de capteur et/ou le jeu de données médicales comprenant des valeurs de mesure d'un appareil de tomographie par impédance électrique et/ou d'un appareil respiratoire, le jeu de données médicales indiquant facultativement un état de sédation du patient, et le schéma de traitement comprenant au moins une instruction spécifique à un processus de sevrage d'un appareil respiratoire, dérivée des données de capteur et facultativement de l'au moins un jeu de données médicales.

2. Unité de coordination (2) selon la revendication 1, **caractérisée en ce que** l'unité de coordination (2) est agencée en outre pour commander au moins l'un des appareils techniques médicaux (3) de telle sorte qu'une mise en oeuvre du schéma de traitement ait lieu et/ou de telle sorte que la mise en oeuvre du schéma de traitement soit soutenue.

3. Unité de coordination (2) selon la revendication 2, **caractérisée en ce que** l'unité de coordination (2) est agencée pour sortir le schéma de traitement, pour recevoir un signal de confirmation et pour commander au moins l'un des appareils techniques médicaux (3) de telle sorte que la mise en oeuvre du schéma de traitement ait lieu ou de telle sorte que la mise en oeuvre du schéma de traitement soit soutenue uniquement lorsque le signal de confirmation est reçu, le schéma de traitement comprenant un diagnostic médical.

4. Unité de coordination (2) selon l'une des revendications précédentes, **caractérisée en ce que** l'unité de coordination (2) est agencée pour déclencher une sortie du schéma de traitement, l'unité de coordination (2) présentant un écran pour la sortie du schéma de traitement.

5. Unité de coordination (2) selon l'une des revendications précédentes, **caractérisée en ce que** l'unité de coordination (2) présente un moyen d'entrée,le moyen d'entrée étant un écran tactile.

6. Unité de coordination (2) selon l'une des revendications précédentes, **caractérisée en ce que** l'unité de coordination (2) est agencée pour soumettre une demande à un service de calcul (9) pour l'évaluation des données de capteur et/ou de l'au moins un jeu de données médicales, et pour recevoir, du service de calcul (9), le schéma de traitement ou les données desquelles l'unité de coordination (2) peut déduire le schéma de traitement, et **en ce que** l'unité de coordination (2) est agencée pour effectuer une consultation de base de données sur une base de données de patients.

7. Unité de coordination (2) selon l'une des revendications précédentes, **caractérisée en ce que** l'au moins une instruction spécifique au processus de sevrage est un temps de départ pour le processus de sevrage, un temps de fin pour le processus de sevrage ou une instruction pour l'ajustement d'un paramètre de ventilation.

8. Unité de coordination (2) selon l'une des revendications précédentes, **caractérisée en ce que** le schéma de traitement comprend une instruction pour le réglage d'une fréquence de ventilation, pour le réglage d'un niveau d'une pression de ventilation et/ou pour le réglage d'un volume tidal pour permettre une respiration spontanée.

9. Système de traitement (1) comprenant une unité de coordination (2) et au moins un appareil technique médical (3), l'unité de coordination (2) étant accouplée à l'appareil technique médical (3) par une connexion de données, **caractérisé en ce que** l'unité de coordination (2) est conçue selon l'une des revendications 1 à 8.

10. Système de traitement (1) selon la revendication 9, **caractérisé en ce que** le système de traitement (1) présente un service de calcul (9), l'unité de coordination (2) étant accouplée au service de calcul (9) par une connexion de données, le service de calcul (9) étant formé par des ordinateurs distribués dans un réseau de communication.

11. Système de traitement (1) selon l'une des revendications 9 ou 10, **caractérisé en ce que** le système de traitement présente un ordinateur de soutien (7), lequel est accouplé à l'unité de coordination (2) par une connexion de données, l'ordinateur de soutien (7) étant agencé pour effectuer des calculs pour l'unité de coordination (2) et/ou pour fournir des jeux de données médicales et/ou pour fournir des données de capteur à l'unité de coordination (2), et l'ordinateur de soutien (7) présentant une base de données.

12. Système de traitement (1) selon la revendication 11, **caractérisé en ce que** l'ordinateur de soutien (7) est agencé pour demander des informations à un système d'information hospitalier (10) par une connexion de données et pour fournir les informations à l'unité de coordination (2), les informations comprenant les données de capteur et/ou les jeux de données médicales.
